# EUROPEAN PATENT SPECIFICATION

(11) **EP 4 437 925 B1**
(45) Date of publication and mention of the grant of the patent: **09.04.2025**
(21) Application number: 22913676.7
(22) Date of filing: 17.10.2022
(51) Int. Cl.: A61B 1/00, H01R 13/502, A61B 1/012, A61B 1/018

(54) **INSERTING APPARATUS, ENDOSCOPE AND USE METHOD**
EINFÜHRVORRICHTUNG, ENDOSKOP UND VERWENDUNGSVERFAHREN
APPAREIL D'INSERTION, ENDOSCOPE ET PROCÉDÉ D'UTILISATION

(30) Priority: 30.12.2021 CN 202111650823
(43) Date of publication of application: 02.10.2024
(73) Proprietor: Hunan Vathin Medical Instrument Co. Ltd, Xiangtan, Hunan 411201 (CN)
(72) Inventor: DAI, Yongyi, Xiangtan, Hunan 411100 (CN)
(74) Representative: Bayramoglu et al.
(86) International application number: PCT/CN2022/125753
(87) International publication number: WO 2023/124412

(56) References cited:
- CN-A- 113 367 646
- CN-A- 113 749 599
- CN-A- 114 287 862
- CN-U- 212 365 886
- CN-U- 212 647 083
- CN-U- 213 782 382
- CN-U- 213 845 800
- DE-A1- 102016 122 864
- JP-A- 2009 039 205
- JP-A- 2013 043 064
- JP-A- 2021 053 022
- JP-B2- 5 154 857
- US-A1- 2019 059 705
- US-A1- 2021 307 596

## Description

### TECHNICAL FIELD

The present disclosure relates to the technical field of endoscopy, and in particular to an inserting apparatus, an endoscope, and a use method.

### BACKGROUND

An endoscope typically includes a rigid or flexible tube, a lighting unit, and an observation structure, and enters the human body through a natural orifice or a small surgical incision. Existing endoscopes are usually disposable and their components are not reusable, resulting in a very high costs of the endoscopes.

Prior art document JP 5 154857 discloses an endoscope system which has a sheath arranged at the peripheral side of an endoscope insertion tube. A cooling device supplies fluid inside the sheath so as to cool the endoscope insertion tube. The sheath is removed by connecting an air supply tube of the cooling device to a base cap. A sheath side gap obstruction wall is formed to fill the gap between a sheath fixed hole of the base cap and the sheath.

### SUMMARY

The invention is defined in the appended claims.

In a first aspect, the invention as defined in claim 1 relates to an inserting apparatus, including a base and a movable element, where
the base is a hollow structure; an inner wall of the base is provided with a step surface; a fixed element is fixedly provided in the base; a gap is formed between one end of the fixed element and the step surface; the gap is configured to accommodate a fixed end of an elastic element; and a pressing element is slidable along an inner wall of the fixed element to push a free end of the elastic element; and
the movable element includes one end connected to a signal line and the other end connected to a support tube; a front end of the support tube is connected to an insertion object, and the support tube is able to pass through the base; and the movable element is at least partially movable along an inner wall of the pressing element to enter the base.

Optionally, the inner wall of the fixed element is provided with a positioning groove, and the pressing element is provided with a positioning protrusion; the positioning groove is configured to accommodate the positioning protrusion; and the positioning protrusion is slidable in the positioning groove.

Optionally, the pressing element, the fixed element, and the elastic element are coaxially arranged.

Optionally, an axial length of the pressing element is greater than an axial length of the fixed element.

Further, according to the invention as defined in claim 1, the elastic element is annular; and the support tube and the movable element are able to pass through the elastic element 40.

Further, according to the invention as defined in claim 1, an end of the pressing element away from the elastic element is provided with a force application portion.

Optionally, the inner wall of the base is further provided with a conical accommodating portion; and the accommodating portion is connected to the step surface and configured to accommodate the elastic element for deformation.

Optionally, the movable element is provided with an electrical connection portion; and the electrical connection portion is connected to an external power source for supplying power to the insertion object.

In a second aspect, a preferred embodiment of the invention provides an endoscope, including a handle device and the inserting apparatus according to the first aspect, where the inserting apparatus is provided on the handle device; and the insertion object at the front end of the support tube is able to enter the handle device.

In a third aspect, the invention as defined in claim 7 relates to a use method of the inserting apparatus according to the first aspect, including the following steps:
S10: pressing, by the force application portion, the pressing element when the movable element and the support tube enter the base; pushing, by the pressing element, the elastic element to deform until the movable element is able to pass through the elastic element; and keeping the pressing element stationary;
S20: allowing the support tube and the movable element to sequentially pass through the pressing element and the elastic element to enter the base;
S30: adjusting the movable element to an appropriate position in the base; stopping pressing on the force application portion; and allowing the elastic element to rebound, so as to clamp the movable element; and
S40: pressing, by the force application portion, the pressing element when the movable element and the support tube leave the base; pushing, by the pressing element, the elastic element to deform until the movable element is movable; and pulling the movable element and the support tube out.

Compared with the prior art, the present disclosure has at least the following technical effects.
(1) In the inserting apparatus provided by the present disclosure, the fixed element, the elastic element, and the pressing element in the base cooperate with each other, such that the insertion object can pass through the base to enter a required component. Due to the axial deformation of the elastic element, the insertion object can be fixed at any desired position in the base, thereby achieving fast and stepless positioning of the insertion object.
(2) In the present disclosure, the elastic element and the pressing element are combined to achieve the positioning of the insertion object at any position, and the design provides a simple structure.
(3) The inserting apparatus is located on the endoscope, and the lighting unit and camera unit are inserted into the endoscope. After the endoscope is used, the handle device can be discarded, while the inserting apparatus carrying the lighting unit and camera unit can be removed for recycling. The design greatly reduces the cost of the endoscope and achieves a simple structure and convenient operation.

### BRIEF DESCRIPTION OF THE DRAWINGS

To describe the technical solutions in the embodiments of the present disclosure more clearly, the following briefly introduces the drawings required for describing the embodiments or the prior art. Apparently, the drawings in the following description show merely some embodiments of the present disclosure, and persons of ordinary skill in the art may still derive other drawings from these drawings without creative efforts.
FIG. 1 is an overall structural diagram of an inserting apparatus according to the present disclosure;
FIG. 2 is a connection diagram of a movable element, a support tube, and a signal line according to the present disclosure;
FIG. 3 is an exploded view of a base, an elastic element, a fixed element, and a pressing element according to the present disclosure;
FIG. 4 is a sectional view taken along A-A shown in FIG. 1;
FIG. 5 is an enlarged view of a position circled in FIG. 4;
FIG. 6 is a structural diagram of the base according to the present disclosure;
FIG. 7 is an axial sectional view of the base according to the present disclosure;
FIG. 8 is a schematic diagram of a locking element of the movable element according to the present disclosure;
FIG. 9 is an axial sectional view of the movable element according to the present disclosure;
FIG. 10 is a structural diagram of the fixed element according to the present disclosure;
FIG. 11 is a structural diagram of the elastic element according to the present disclosure;
FIG. 12 is a structural diagram of the pressing element according to the present disclosure;
FIG. 13 is a structural diagram of an electrical connection portion according to the present disclosure; and
FIG. 14 is a structural diagram of an endoscope according to the present disclosure.

Reference Numerals: 1. inserting apparatus; 10. base; 11. step surface; 12. accommodating portion; 13. access hole; 14. electrical connection slot; 20. movable element; 21. electrical connection portion; 22. locking element; 23. support element; 30. fixed element; 31. positioning groove; 32. protrusion; 40. elastic element; 41. outer edge; 42. inner edge; 43. through-hole; 50. pressing element; 51. positioning protrusion; 52. force application portion; 60. signal line; 70. support tube; 80. electrical connection portion; 81. first electrical connection terminal; 82. second electrical connection terminal; and 2. handle device.

### DETAILED DESCRIPTION OF THE EMBODIMENTS

The following description provides many different embodiments or examples for implementing different features of the present disclosure. The elements and arrangements described in the following specific examples are only intended to concisely express the present disclosure, and are only for illustration purposes, rather than to limit the present disclosure.

In order to make the objectives, technical solutions, and advantages of the embodiments of the present disclosure clearer, the technical solutions in the embodiments of the present disclosure will be clearly and completely described below in conjunction with the drawings in the embodiments of the present disclosure. Apparently, the described embodiments are some, rather than all of the embodiments of the present disclosure. Generally, components of the embodiments of the present disclosure described and shown in the drawings may be arranged and designed in various manners.

Therefore, the following detailed description of the embodiments of the present disclosure in the drawings is not intended to limit the protection scope of the present disclosure, but merely indicates selected embodiments of the present disclosure. All other embodiments obtained by those of ordinary skill in the art based on the embodiments of the present disclosure without creative efforts should fall within the protection scope of the present disclosure.

It should be noted that similar reference signs and letters represent similar items in the drawings below. Therefore, once an item is defined in one drawing, it does not need to be further defined and described in subsequent drawings.

In the description of the present disclosure, it should be noted that orientations or position relationships indicated by terms such as "upper", "lower", "inner", and "outer" are orientation or position relationships shown in the drawings, and these terms are only used to facilitate description of the present disclosure and simplify the description, but not to indicate or imply that the mentioned apparatus or components must have a specific orientation or must be established and operated in a specific orientation, and thus these terms cannot be understood as a limitation to the present disclosure.

In addition, the terms such as "first" and "second" are used only for distinguishing description and cannot be understood as indicating or implying relative importance.

It should be noted that, if there is no conflict, the following embodiments and features in the embodiments of the present disclosure may be mutually combined.

### Embodiment 1

Embodiment 1 of the present disclosure provides inserting apparatus 1, including base 10 and movable element 20.

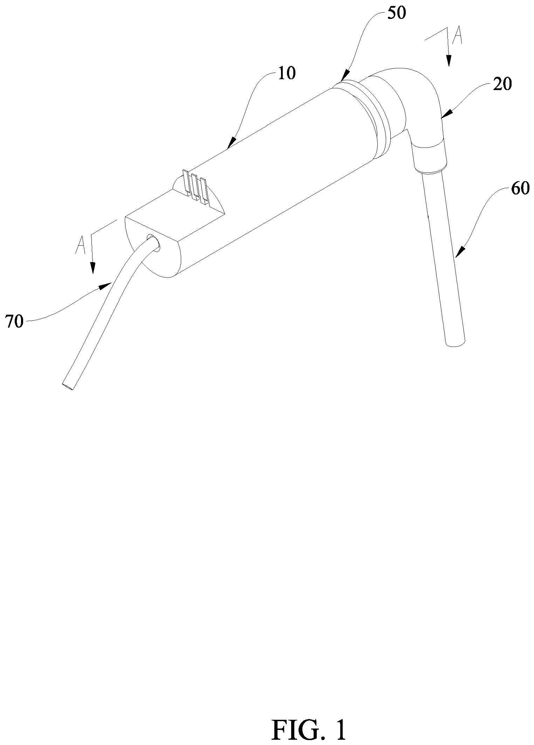

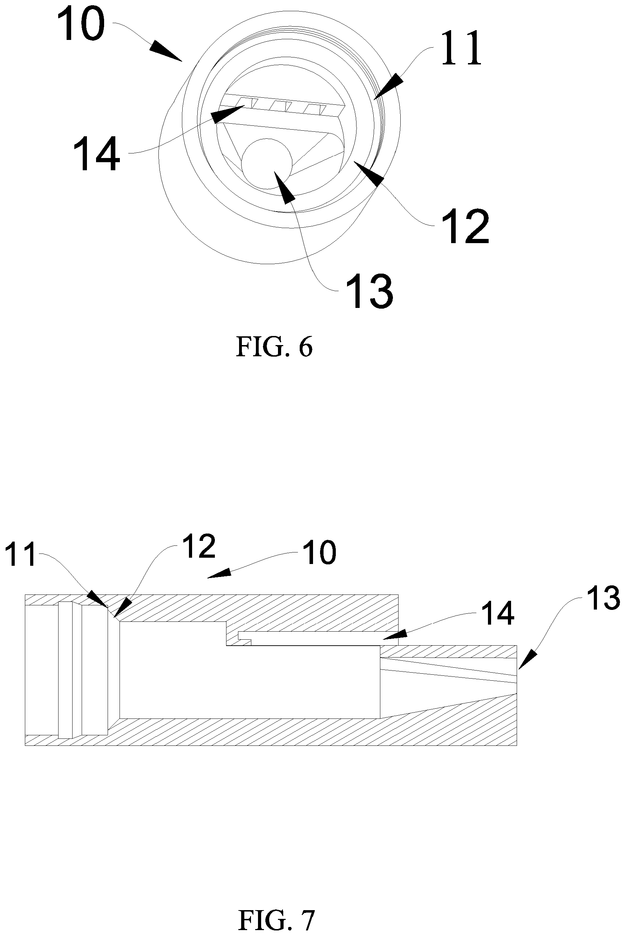

As shown in FIGS. 1 to 14, the base 10 is a hollow structure. An inner wall of the base 10 is provided with step surface 11. Fixed element 30 is fixedly provided in the base 10. A gap is formed between one end of the fixed element 30 and the step surface 11. The gap is configured to accommodate a fixed end of elastic element 40. Pressing element 50 is slidable along an inner wall of the fixed element 30 to push a free end of the elastic element 40.

The elastic element 40 has a closed shape, such as a circle, a triangle, a square or a diamond, which is not limited herein. The fixed end of the elastic element 40 is an end located in the gap, and the free end of the elastic element is an end away from the gap. In this embodiment, the fixed end of the elastic element 40 forms outer edge 41, and the free end of the elastic element forms inner edge 42.

The movable element 20 includes one end connected to signal line 60 and the other end connected to support tube 70. A front end of the support tube 70 is connected to an insertion object, and the support tube 70 is able to pass through the base 10. The movable element 20 is at least partially movable along an inner wall of the pressing element 50 to enter the base 10.

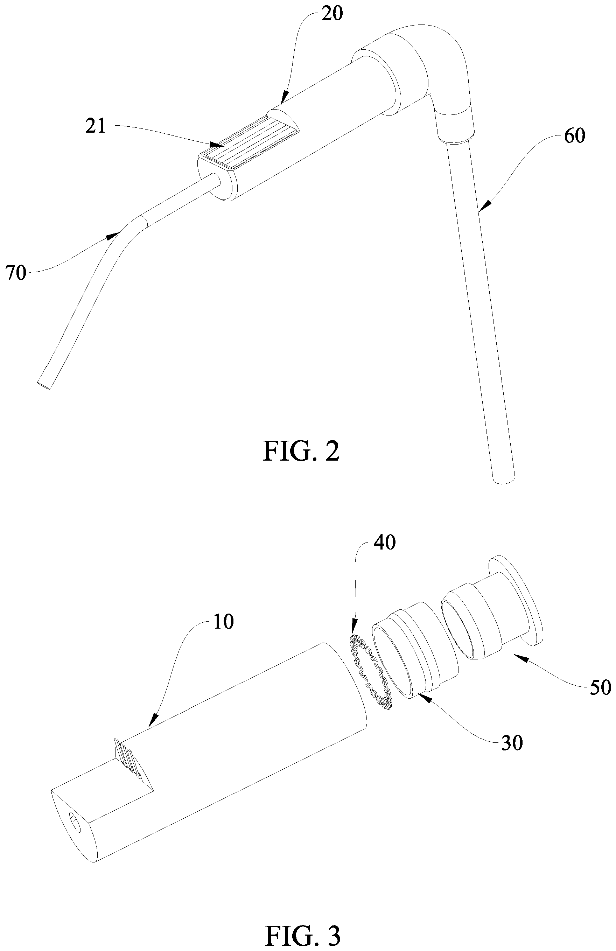

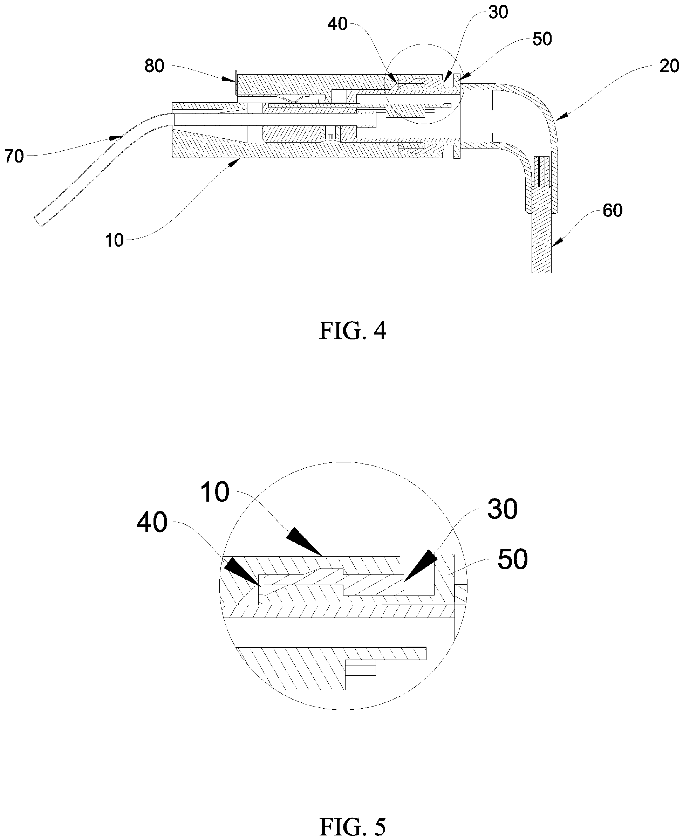

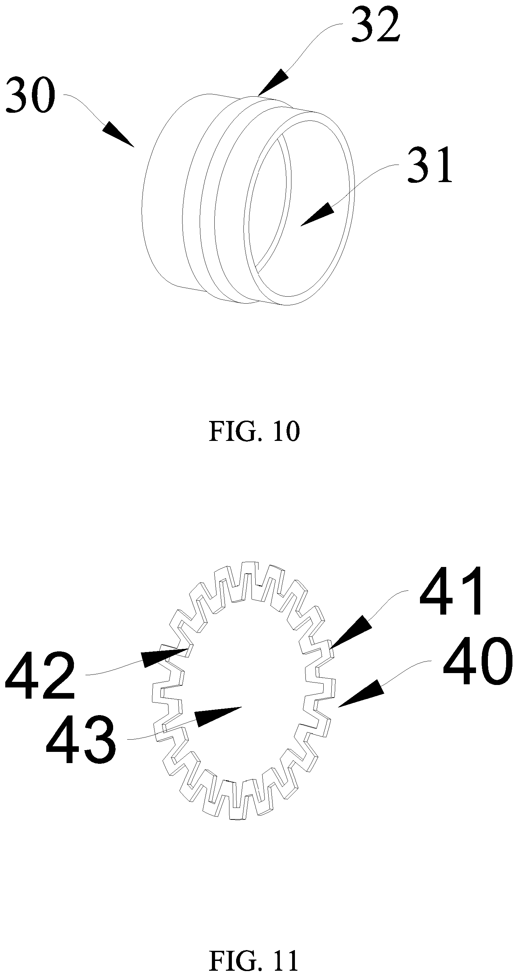

In the above solution, as shown in FIG. 1, the base 10 is overall cylindrical. The base 10 can further be in the shape of a rectangular solid, a cuboid or a prism, which is not limited herein. The base 10 is a hollow structure. The inner wall of the base 10 is provided with the step surface 11. The step surface 11 is provided at one end close to the pressing element 50. As shown in FIGS. 3 and 4, the fixed element 30 is adapted to the shape of the base 10, and the fixed element 30 is also a hollow structure. The fixed element 30 is fixedly provided in the base 10. One end of the fixed element 30 is close to the step surface 11. It does not contact with the step surface 11, but forms a gap with the step surface. The gap accommodates the elastic element 40. It can be understood that one side of the elastic element 40 is in contact with the step surface 11, while the other side of the elastic element is in contact with the fixed element 30. The elastic element 40 is not fixedly connected to any component. If the elastic element 40 is damaged, it can be easily disassembled and replaced, and the design also facilitates partial deformation of the elastic element 40. A thickness of elastic element 40 is less than or equal to a width of the gap. The width of the gap is dynamically adjustable according to the thickness of the elastic element 40 so as to adapt to different thicknesses of the elastic element 40 for different needs. The pressing element 50 is located inside the fixed element 30. When it is necessary to insert the external insertion object into the base 10, the pressing element 50 is pressed by an external force. That is, an axial force is applied to the pressing element 50, causing the pressing element 50 to slide along the inner wall of the fixed element 30, as shown in FIG. 11. A front end of the pressing element 50 is abutted against the inner edge 42 of the elastic element 40, and the inner edge 42 is pushed to deform axially, causing a diameter of through-hole 43 to increase. The support tube 70 and a portion of the movable element 20 can sequentially enter the base 10 through the pressing element 50 and the through-hole 43 of the elastic element 40, such that the insertion object is inserted into an inserting unit through the base 10.

As shown in FIG. 2, one end of the movable element 20 is connected to the signal line 60. It can be understood that the one end of movable element 20 is directly connected to the signal line or the one end of movable element 20 is connected to a signal tube holding the signal line, which is not limited herein. The other end of the movable element is connected to the support tube 70. The front end of the support tube 70 is connected to the insertion object. The support tube 70 is configured to support the insertion object, in order to smoothly deliver the insertion object to a designated position or pull out and recover the insertion object for multiple uses.

It should be noted that the insertion object can be a camera or a light-emitting diode (LED) in the field of endoscopy, or it can be a device component that needs to be reused in other fields.

Optionally, the movable element 20, the signal line 60, and the support tube 70 can be connected by means such as welding, integrated molding, adhesive bonding, threading or clamping, which is not limited herein.

Optionally, the fixed element 30 and the base 10 can be connected through internal and external threads or embedded clamping, and can be fixedly connected by means such as integrated molding, adhesive bonding or welding, which is not limited herein.

Therefore, in the inserting apparatus 1 provided by the present disclosure, the fixed element 30, the elastic element 40, and the pressing element 50 in the base 10 cooperate with each other, such that the insertion object can pass through the base 10 to enter a required component. Due to the axial deformation of the elastic element 40, the insertion object can be fixed at any desired position in the base 10, thereby achieving fast and stepless positioning of the insertion object.

Further, the inner wall of the fixed element 30 is provided with positioning groove 31, and the pressing element 50 is provided with positioning protrusion 51. The positioning groove 31 is configured to accommodate the positioning protrusion 51, and the positioning protrusion 51 is slidable in the positioning groove 31.

In the above solution, as shown in FIGS. 5, 10, and 12, the fixed element 30 is a hollow structure. The inner wall of the fixed element 30 is provided with the positioning groove 31. The positioning groove 31 is provided at an end close to the elastic element 40. An outer wall of the pressing element 50 is provided with the positioning protrusion 51 corresponding to the positioning groove 31. The front end of the pressing element 50 is provided with a reduced portion. When an external force is applied to push the pressing element 50, the positioning protrusion 51 is slidable along the positioning groove 31. The reduced portion is abutted against the inner edge 42 of the elastic element 40 and is able to push the elastic element 40 to deform axially. When the insertion object reaches an appropriate position, the external force applied to the pressing element 50 is removed. The elastic element 40 contracts to lock the movable element 20, causing the movable element 20 to stop moving in the base 10.

The positioning groove 31 in the fixed element 30 axially positions the pressing element 50. Otherwise, when the external force applied to the pressing element 50 remains unchanged or disappears, the pressing element 50 will be subjected to a rebound force of the elastic element 40, causing the pressing element 50 to detach from the fixed element 30, such that the elastic element 40 cannot lock the movable element 20.

As shown in FIG. 10, protrusion 32 is provided on a surface of the fixed element 30. The base 10 is provided with a groove corresponding to the protrusion 32. The protrusion 32 and the groove 31 are clamped to axially position the fixed element 30 in the base 10, avoiding axial movement of the fixed element 30 when the pressing element 50 moves in the fixed element 30.

Further, the pressing element 50, the fixed element 30, and the elastic element 40 are coaxially arranged.

In the above solution, as shown in FIGS. 4 and 5, when the support tube 70 and the portion of the movable element 20 enter the base 10, the pressing element 50 is sleeved on the movable element 20, the fixed element 30 is sleeved on the pressing element 50, and the elastic element 40 is located in a front section of the fixed element 30 and the pressing element 50 and sleeved on the movable element 20. The pressing element 50, the fixed element 30, and the elastic element 40 are coaxially arranged such that the movable element 20, the support tube 70 and the insertion object can smoothly enter and pass through the base 10.

Further, an axial length of the pressing element 50 is greater than an axial length of the fixed element 30.

In the above solution, as shown in FIGS. 1, 4, and 5, the axial length of the pressing element 50 is greater than the axial length of the fixed element 30, such that the pressing element 50 has a sufficient length to slide relative to the fixed element 30 and an appropriate length to push the elastic element 40, causing the elastic element to make required axial deformation. If the length of the pressing element 50 is equal to or less than the length of the fixed element 30, it is not easy to push the pressing element 50 to slide through an external force, and the axial deformation of the elastic element 40 cannot reach an actual required amount.

It should be noted that the length of the pressing element 50 only needs to be greater than the length of the fixed element 30. The specific length of the pressing element 50 can be set according to actual needs, and it is not limited herein.

Further, the elastic element 40 is annular, and the support tube 70 and the movable element 20 can pass through the elastic element 40.

In the above solution, as shown in FIGS. 11 and 4, the elastic element 40 includes the outer edge 41, the inner edge 42, and the through hole 43. A zone enclosed by the outer edge 41 and the inner edge 42 includes hollow teeth. When the outer edge 41 is located in the base 10, the outer edge 41 is located in the gap formed by the step surface 11 and the one end of the fixed element 30 and is in close contact with the step surface and the one end of the fixed element, and one side of the inner edge 42 is abutted against the pressing element 50. When the pressing element 50 pushes the inner edge 42 to move axially, the outer edge 41 remains stationary in the gap, the inner edge 42 deforms axially, and a diameter of the through-hole 43 increases. When the diameter of the through-hole 43 is greater than a diameter of the movable element 20, the support tube 70 and the movable element 20 pass through the through-hole 43 and enter the base 10. When the insertion object connected to the front end of the support tube 70 reaches an appropriate position, the pressing element 50 is not pushed any more, and the inner edge 42 rebounds axially. The diameter of the through-hole 43 decreases to lock the movable element 20.

Further, an end of the pressing element 50 away from the elastic element 40 is provided with force application portion 52.

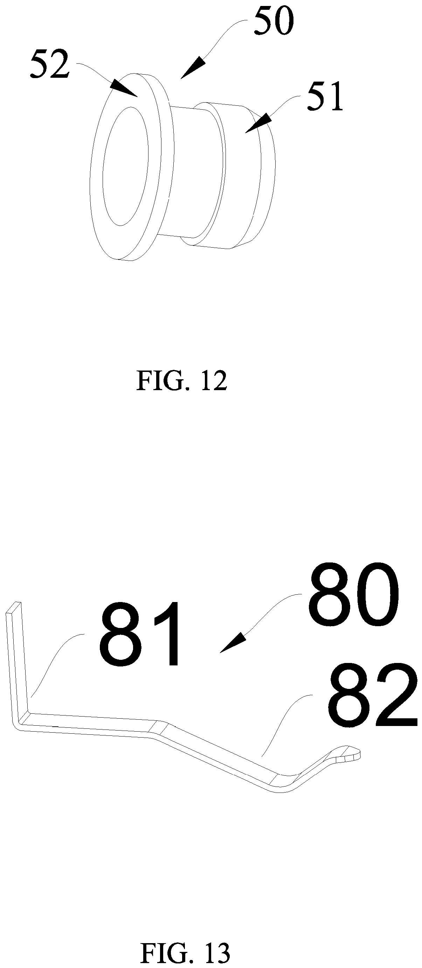

In the above solution, as shown in FIG. 12, one end of the pressing element 50 is provided with the force application portion 52. A diameter of the force application portion 52 is larger than a diameter of the fixed element 30, and a groove is formed between the force application portion 52 and the positioning protrusion 51. The pressing element 30 is provided with a protrusion corresponding to the groove. The protrusion and the groove are matched to achieve axial positioning of the pressing element 50, avoiding excessive force application during a pressing process to press the force application portion 52 of the pressing element 50 into the fixed element 30.

It should be noted that an object of applying a force to the force application portion 52 can be an operator or a tool, which is not limited herein.

Further, the inner wall of the base 10 is further provided with accommodating portion 12. The accommodating portion 12 is conical. The accommodating portion 12 is connected to the step surface 11, and the accommodating portion 12 is configured to accommodate the elastic element 40 for deformation.

In the above solution, as shown in FIGS. 5, 6, and 7, the accommodating portion 12 is located below the step surface 11. The outer edge 41 of the elastic element 40 is located in the gap. One side of the inner edge 42 is abutted against the pressing element 50, and the other side of the inner edge forms an accommodating space with the accommodating portion 12. When the pressing element 50 pushes the inner edge 42, the inner edge 42 undergoes a certain degree of deformation in the accommodating space. The accommodating portion 12 ensures that the inner edge 42 undergoes deformation in the space of a combined force, avoiding the inability of the inner edge to recover due to excessive deformation or the inability of the inner edge to achieve an expected effect due to small deformation.

The base 10 is further provided with access hole 13. After entering the base 10, the support tube 70 passes through the access hole 13 to enter a required insertion component.

Further, the movable element 20 is provided with electrical connection portion 21. The electrical connection portion 21 is connected to an external power source for supplying power to the insertion object.

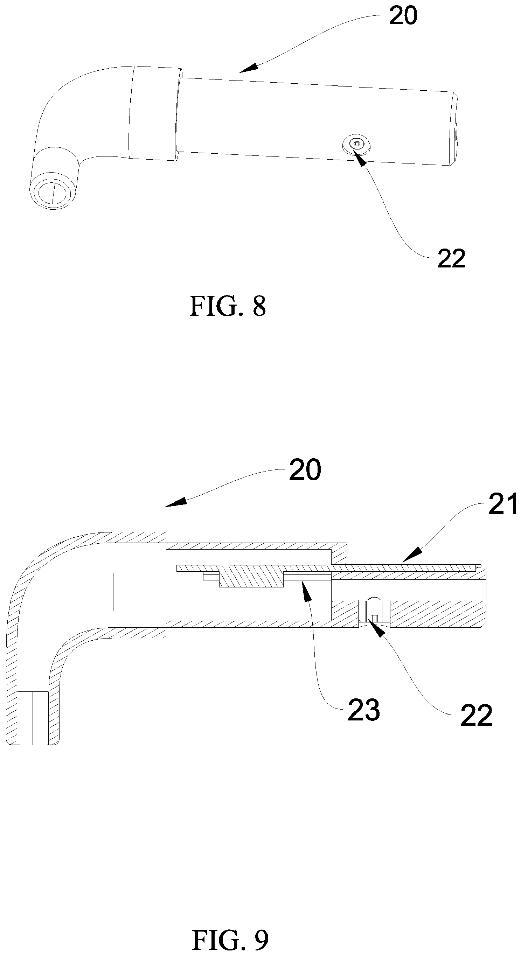

In the above solution, as shown in FIGS. 2 and 9, the electrical connection portion 21 is provided at one end of the movable element 20 close to the support tube 70. The electrical connection portion 21 is provided with an electrode plate. The electrode plate is fixedly provided in the movable element 20 and electrically connected to the external power source for supplying power to the insertion object.

Optionally, the movable element 20 is cylindrical. A cylindrical outer wall is provided with a flat surface. The step surface is formed between the flat surface and the outer wall. The electrode plate is located on the flat surface. As shown in FIGS. 4 and 1, correspondingly, the base 10 is further provided with a flat surface and a step surface. When the movable element 20 enters the base 10, the step surface is abutted against the inner wall of the base 10 to axially position the movable element 20. The flat surface circumferentially positions the movable element 20 in the base 10 to avoid rotation and movement of the insertion object at the front end of the support tube 70 during use.

As shown in FIG. 2, the movable element 20 includes two portions with different diameters. The diameter of the portion close to the signal line is greater than the diameter of the other portion. These two portions form a step surface at a diameter change position. When the movable element 20 enters the base 10, the step surface is abutted against the pressing element 50 to avoid the position of the insertion object beyond a target position due to the excessive entry of the movable element 20 into the base 10.

In this embodiment, as shown in FIGS. 4, 6, 7, and 13, electrical connection portion 80 is provided. The electrical connection portion 80 is fixed in electrical connection slot 14. The electrical connection slot 14 is in an "L" shape. The electrical connection portion 80 includes first electrical connection terminal 81 and second electrical connection terminal 82. The first electrical connection terminal 81 is configured to connect the external power source, and the second electrical connection terminal 82 is configured to abut against the electrode plate of the electrical connection portion 21. The second electrical connection terminal 82 is axially slidable along the electrode plate and maintains an electrical connection between the electrical connection portion 21 of the movable element 20 and the electrical connection portion 80 within a movable range, thereby ensuring that the insertion object is in a charged state.

Specifically, the first electrical connection portion 81 is in an "L" shape, which facilitates its mounting in the electrical connection slot 14. The second electrical connection portion 82 is in a "V" shape, and includes one end connected to the first electrical connection portion 81, a middle portion abutted against the electrical connection portion 21, and the other end abutted against an inner wall of the electrical connection slot 14. A length of the electrical connection slot 14 is greater than a length of the second electrical connection portion 82.

Optionally, there may be one, two, or three electrical connection portions 80, which is not limited herein. In this embodiment, preferably, there are three electrical connection portions.

As shown in FIGS. 8 and 9, an inner wall of the movable element 20 is symmetrically provided with support element 23 for supporting the electrical connection portion 21 and limiting the electrical connection portion 21 within an appropriate range. The movable element 20 is provided with locking element 22. When the support tube 70 is in an appropriate position within the movable element 20, the locking element 22 fixes and locks the support tube 70, such that the support tube 70 is fixedly provided in the movable element 20.

### Embodiment 2

Embodiment 2 of the present disclosure provides an endoscope, including handle device 2 and the inserting apparatus 1 described in Embodiment 1. The inserting apparatus 1 is provided on the handle device 2, and the insertion object at the front end of the support tube 70 is able to enter the handle device 2.

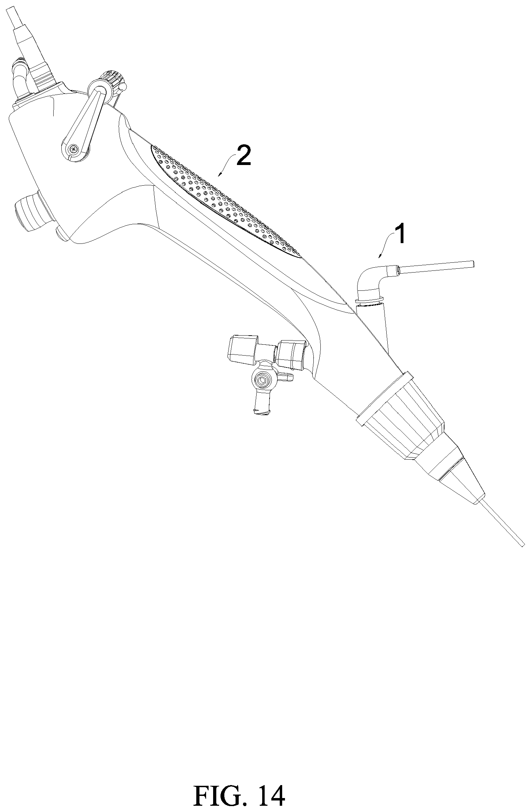

In the above solution, as shown in FIG. 14, in this embodiment, the inserting apparatus 1 is provided on the handle device 2 of the endoscope. The base 10 of the inserting apparatus 1 is detachably provided on the handle device 2 or provided on the handle device 2 through integrated molding. The insertion object can be a camera unit, a lighting unit, or other element that can be inserted separately. When an external force is applied to press the pressing element 50, the elastic element 40 is pushed to deform. The diameter of through-hole 43 increases, and the support tube 70 passes through the base 10 and enters the handle device 2. The position of the support tube 70 in the handle device 2 is adjusted. When the insertion object reaches an appropriate position in a front-end element, the pressing element 50 is not pressed any more. The elastic element 40 locks the movable element 20 and locks the support tube 70 and the insertion object. The front end of the front-end element is provided with sealed glass to prevent contamination of the insertion object during use. After the endoscope is used, the pressing element 50 is pressed to stop the elastic element 40 from locking the movable element 20, and the support tube 70 is pulled out of the handle device 2 for next use.

### Embodiment 3

The third aspect of the present disclosure provides a use method of the inserting apparatus 1 described in Embodiment 1, including the following steps.

S10. When the movable element 20 and the support tube 70 enter the base 10, the pressing element 50 is pressed by the force application portion 52. The pressing element 50 pushes the elastic element 40 to deform until the movable element 20 is able to pass through the elastic element 40, and the pressing element 50 keeps stationary.

S20. The support tube 70 and the movable element 20 sequentially pass through the pressing element 50 and the elastic element 40, and enter the base 10.

S30. The movable element 20 is adjusted to an appropriate position in the base 10. The pressing on the force application portion 52 is stopped, and the elastic element 40 rebounds to clamp the movable element 20.

S40. When the movable element 20 and the support tube 70 leave the base 10, the pressing element 50 is pressed by the force application portion 52. The pressing element 50 pushes the elastic element 40 to deform until the movable element 20 is movable, and the movable element 20 and the support tube 70 are pulled out.

The above are merely preferred embodiments of the present disclosure, and not intended to limit the present disclosure. The invention is defined in the appended claims.

## Claims

1. An inserting apparatus (1) comprising a base (10) and a movable element (20), wherein
the base (10) is a hollow structure; an inner wall of the base (10) is provided with a step surface (11); a fixed element (30) is fixedly provided in the base (10); a gap is formed between one end of the fixed element (30) and the step surface (11); the gap is configured to accommodate a fixed end of an elastic element (40); and a pressing element (50) is slidable along an inner wall of the fixed element (30) to push a free end of the elastic element (40);
the movable element (20) comprises a first end connected to a signal line (60) and a second end connected to a support tube (70); a front end of the support tube (70) is connected to an insertion object, and the support tube (70) is allowed to pass through the base (10); and the movable element (20) is at least partially movable along an inner wall of the pressing element (50) to enter the base (10); and
the elastic element (40) is annular; the support tube (70) and the movable element (20) are allowed to pass through the elastic element (40); and an end of the pressing element (50) away from the elastic element (40) is provided with a force application portion (52).

2. The inserting apparatus (1) according to claim 1, **characterized in that** the inner wall of the fixed element (30) is provided with a positioning groove (31), and the pressing element (50) is provided with a positioning protrusion (51); the positioning groove (31) is configured to accommodate the positioning protrusion (51); and the positioning protrusion (51) is slidable in the positioning groove (31).

3. The inserting apparatus (1) according to claim 1, **characterized in that** the pressing element (50), the fixed element (30), and the elastic element (40) are coaxially arranged.

4. The inserting apparatus (1) according to one of claims 1 to 3, **characterized in that** an axial length of the pressing element (50) is greater than an axial length of the fixed element (30).

5. The inserting apparatus (1) according to claim 1, **characterized in that** the inner wall of the base (10) is further provided with a conical accommodating portion (12); and the accommodating portion (12) is connected to the step surface (11) and configured to accommodate the free end of the elastic element (40).

6. The inserting apparatus (1) according to claim 1, **characterized in that** the movable element (20) is provided with an electrical connection portion (21) for supplying power to the insertion object.

7. A use method of the inserting apparatus (1) according to one of claims 1 to 6, **characterized by** comprising the following steps:
S10: pressing the pressing element (50) when the movable element (20) and the support tube (70) enter the base (10); pushing, by the pressing element (50), the elastic element (40) to deform until the movable element (20) is allowed to pass through the elastic element (40); and keeping the pressing element (50) stationary;
S20: allowing the support tube (70) and the movable element (20) to sequentially pass through the pressing element (50) and the elastic element (40) to enter the base (10);
S30: adjusting the movable element (20) to an appropriate position in the base (10); stopping the pressing on the pressing element (50); and allowing the elastic element (40) to rebound, so as to clamp the movable element (20); and
S40: pressing the pressing element (50) when the movable element (20) and the support tube (70) leave the base (10); pushing, by the pressing element (50), the elastic element (40) to deform until the movable element (20) is movable; and pulling the movable element (20) and the support tube (70) out.

8. An endoscope, comprising a handle device (2) and the inserting apparatus (1) according to one of claims 1 to 6, **characterized in that** the inserting apparatus (1) is provided on the handle device (2); and the insertion object at the front end of the support tube (70) is allowed to enter the handle device (2).

## Patentansprüche

1. Einsetzgerät (1), das eine Basis (10) und ein bewegbares Element (20) umfasst, wobei
die Basis (10) eine hohle Struktur ist; eine Innenwand der Basis (10) mit einer Stufenfläche (11) versehen ist; ein feststehendes Element (30) feststehend in der Basis (10) bereitgestellt ist; eine Lücke zwischen einem Ende des feststehenden Elements (30) und der Stufenfläche (11) gebildet ist; die Lücke dazu konfiguriert ist, ein feststehendes Ende eines elastischen Elements (40) aufzunehmen; und ein Druckelement (50) entlang einer Innenwand des feststehenden Elements (30) verschiebbar ist, um ein freies Ende des elastischen Elements (40) zu drücken;
das bewegbare Element (20) ein erstes Ende umfasst, das mit einer Signalleitung (60) verbunden ist, und ein zweites Ende, das mit einem Tragrohr (70) verbunden ist; ein vorderes Ende des Tragrohrs (70) mit einem Einsetzobjekt verbunden ist, und es dem Tragrohr (70) erlaubt ist, durch die Basis (10) durchzugehen; und das bewegbare Element (20) mindestens teilweise entlang einer Innenwand des Druckelements (50) bewegbar ist, um in die Basis (10) einzutreten; und
das elastische Element (40) ringförmig ist; es dem Tragrohr (70) und dem bewegbaren Element (20) erlaubt ist, durch das elastische Element (40) durchzugehen; und ein Ende des Druckelements (50) entfernt von dem elastischen Element (40) mit einem Kraftanwendungsabschnitt (52) versehen ist.

2. Einsetzgerät (1) nach Anspruch 1, **dadurch gekennzeichnet, dass** die Innenwand des feststehenden Elements (30) mit einer Positionierungsnut (31) versehen ist, und das Druckelement (50) mit einem Positionierungsvorsprung (51) versehen ist; die Positionierungsnut (31) dazu konfiguriert ist, den Positionierungsvorsprung (51) aufzunehmen; und der Positionierungsvorsprung (51) in der Positionierungsnut (31) verschiebbar ist.

3. Einsetzgerät (1) nach Anspruch 1, **dadurch gekennzeichnet, dass** das Druckelement (50), das feststehende Element (30) und das elastische Element (40) koaxial eingerichtet sind.

4. Einsetzgerät (1) nach einem der Ansprüche 1 bis 3, **dadurch gekennzeichnet, dass** eine axiale Länge des Druckelements (50) größer als eine axiale Länge des feststehenden Elements (30) ist.

5. Einsetzgerät (1) nach Anspruch 1, **dadurch gekennzeichnet, dass** die Innenwand der Basis (10) weiter mit einem konischen Aufnahmeabschnitt (12) versehen ist; und der Aufnahmeabschnitt (12) mit der Stufenfläche (11) verbunden und dazu konfiguriert ist, das freie Ende des elastischen Elements (40) aufzunehmen.

6. Einsetzgerät (1) nach Anspruch 1 **dadurch gekennzeichnet, dass** das bewegbare Element (20) mit einem elektrischen Verbindungsabschnitt (21) zum Zuführen von Leistung zu dem Einsetzobjekt versehen ist.

7. Verwendungsverfahren des Einsetzgeräts (1) nach einem der Ansprüche 1 bis 6, **dadurch gekennzeichnet, dass** es die folgenden Schritte umfasst:
S10: Drücken des Druckelements (50), wenn das bewegbare Element (20) und das Tragrohr (70) in die Basis (10) eintreten; Drücken, durch das Druckelement (50), des elastischen Elements (40) zum Verformen, bis es dem bewegbaren Element (20) erlaubt wird, durch das elastische Element (40) durchzugehen; und Stationärhalten des Druckelements (50);
S20: Erlauben, dass das Tragrohr (70) und das bewegbare Element (20) nacheinander durch das Druckelement (50) und das elastische Element (40) durchgehen, um in die Basis (10) einzutreten;
S30: Einstellen des bewegbaren Elements (20) auf eine geeignete Position in der Basis (10); Stoppen des Drückens auf das Druckelement (50); und Erlauben, dass das elastische Element (40) zurückprallt, um das bewegbare Element (20) zu klemmen; und
S40: Drücken des Druckelements (50), wenn das bewegbare Element (20) und das Tragrohr (70) die Basis (10) verlassen; Drücken, durch das Druckelement (50), des elastischen Elements (40) zum Verformen, bis das bewegbare Element (20) bewegbar ist; und Herausziehen des bewegbaren Elements (20) und des Tragrohrs (70).

8. Endoskop, das eine Griffvorrichtung (2) und das Einsetzgerät (1) nach einem der Ansprüche 1 bis 6 umfasst, **dadurch gekennzeichnet, dass** das Einsetzgerät (1) an der Griffvorrichtung (2) bereitgestellt ist; und es dem Einsetzobjekt an dem vorderen Ende des Tragrohrs (70) erlaubt wird, in die Griffvorrichtung (2) einzutreten.

## Revendications

1. Appareil enfichable (1) comprenant une base (10) et un élément mobile (20), dans lequel
la base (10) est une structure creuse ; une paroi interne de la base (10) est pourvue d'une surface étagée (11) ; un élément fixe (30) est monté à demeure dans la base (10) ; un espace est formé entre une extrémité de l'élément fixe (30) et la surface étagée (11) ; l'espace est configuré pour recevoir une extrémité fixe d'un élément élastique (40) ; et un élément de pression (50) est coulissant le long d'une paroi interne de l'élément fixe (30) pour pousser une extrémité libre de l'élément élastique (40) ;
l'élément mobile (20) comprend une première extrémité reliée à une ligne de signal (60) et une seconde extrémité reliée à un tube de support (70) ; une extrémité avant du tube de support (70) est reliée à un objet enfiché et le tube de support (70) est autorisé à passer à travers la base (10) ; et l'élément mobile (20) est mobile au moins partiellement le long d'une paroi interne de l'élément de pression (50) pour entrer dans la base (10) ; et
l'élément élastique (40) est annulaire ; le tube de support (70) et l'élément mobile (20) sont autorisés à passer à travers l'élément élastique (40) ; et une extrémité de l'élément de pression (50) à distance de l'élément élastique (40) est pourvue d'une partie d'application de force (52).

2. Appareil enfichable (1) selon la revendication 1, **caractérisé en ce que** la paroi interne de l'élément fixe (30) est pourvue d'une rainure de positionnement (31) et l'élément de pression (50) est pourvu d'une saillie de positionnement (51) ; la rainure de positionnement (31) est configurée pour recevoir la saillie de positionnement (51) ; et la saillie de positionnement (51) est coulissante dans la rainure de positionnement (31).

3. Appareil enfichable (1) selon la revendication 1, **caractérisé en ce que** l'élément de pression (50), l'élément fixe (30) et l'élément élastique (40) sont disposés coaxialement.

4. Appareil enfichable (1) selon l'une des revendications 1 à 3, **caractérisé en ce qu'**une longueur axiale de l'élément de pression (50) est supérieure à une longueur axiale de l'élément fixe (30).

5. Appareil enfichable (1) selon la revendication 1, **caractérisé en ce que** la paroi interne de la base (10) est en outre pourvue d'une partie de réception conique (12) ; et la partie de réception conique (12) est reliée à la surface étagée (11) et configurée pour recevoir l'extrémité libre de l'élément élastique (40).

6. Appareil enfichable (1) selon la revendication 1, **caractérisé en ce que** l'élément mobile (20) est pourvu d'une partie de connexion électrique (21) pour alimenter l'objet enfiché.

7. Procédé d'utilisation de l'appareil enfichable (1) selon l'une des revendications 1 à 6, **caractérisé en ce qu'**il comprend les étapes suivantes :
S10 : presser l'élément de pression (50) lorsque l'élément mobile (20) et le tube de support (70) entrent dans la base (10) ; pousser, par l'élément de pression (50), l'élément élastique (40) pour se déformer jusqu'à ce que l'élément mobile (20) puisse passer à travers l'élément élastique (40) ; et maintenir l'élément de pression (50) stationnaire ;
S20 : permettre au tube de support (70) et à l'élément mobile (20) de passer de manière séquentielle à travers l'élément de pression (50) et l'élément élastique (40) pour entrer dans la base (10) ;
S30 : ajuster l'élément mobile (20) à une position appropriée dans la base (10) ; stopper la pression sur l'élément de pression (50) ; et permettre à l'élément élastique (40) de rebondir, de manière à serrer l'élément mobile (20) ; et
S40 : presser l'élément de pression (50) lorsque l'élément mobile (20) et le tube de support (70) quittent la base (10) ; pousser, à l'aide de l'élément de pression (50), l'élément élastique (40) pour se déformer jusqu'à ce que l'élément mobile (20) soit mobile ; et tirer l'élément mobile (20) et le tube de support (70) vers l'extérieur.

8. Endoscope, comprenant un dispositif de poignée (2) et l'appareil enfichable (1) selon l'une des revendications 1 à 6, **caractérisé en ce que** l'appareil enfichable (1) est disposé sur le dispositif de poignée (2) ; et l'objet enfiché à l'extrémité avant du tube de support (70) est autorisé à entrer dans le dispositif de poignée (2).
